# EUROPEAN PATENT APPLICATION

(11) **EP 3 588 076 A1**
(43) Date of publication of application: **01.01.2020**
(21) Application number: 18757680.6
(22) Date of filing: 19.02.2018
(51) Int. Cl.: G01N 27/414, G01N 27/416

(54) **ION SENSITIVE BIOSENSOR**

(30) Priority: 21.02.2017 JP 2017029685
(71) Applicant: Provigate Inc., Tokyo 113-0033 (JP)
(72) Inventor: KAJISA, Taira, Tokyo 113-0033 (JP); YANAGIMOTO, Yoshiyuki, Tokyo 113-0033 (JP)
(74) Representative: Weickmann & Weickmann PartmbB
(86) International application number: PCT/JP2018/005685
(87) International publication number: WO 2018/155370

(57) **Abstract**

The object of the present invention is to remove measurement noise in an ion-sensitive biosensor. The present invention provides an ion-sensitive biosensor, characterized in that all or a part of the ion-sensitive electrode surface is coated with polycatecholamine.

## Description

### Technical Field

The present invention relates to an ion-sensitive biosensor comprising an ion-sensitive electrode.

### Background Art

In recent years, research of biosensors that employ Field Effect Transistor (FET) as one method of electrochemical measurement has become active. With a biosensor employing FET, when the sensor recognizes the biomolecule to be the measurement target, physical changes such as heat, mass, and charge as well as chemical changes such as degradation of target substance or generation of the substance occur, these changes are transformed into electric signals with FET which is a signal transformation element as change in charge or change in capacitance, and the target substance is measured. A biosensor employing FET have characteristics such as (1) charges inherent to ions or molecules can be electrically detected, (2) no effort and time is necessary for measurement, (3) real-time measurement is possible, (4) electrical measurement without labeling and without invasion is possible, and (5) downsizing and integration are possible due to semiconductor microproduction technology.

For example, a biosensor that detects DNA hybridization by extended gate FET (Non-Patent Literature 1) or a biosensor that detects various sugars by extended gate FET (Non-Patent Literature 2) and the like have been proposed in the past.

As an ion-sensitive biosensor that employs FET, for example a hydrogen ion-sensitive sensor that utilizes the equilibrium reaction of the terminal carboxyl or amino group of an alkyl group that is bound to a gold gate electrode by thiol bond with a hydrogen ion has been proposed (Non-Patent Literature 3).

### Citation List

[Non-Patent Literature 1] T. Sakata et al., Jpn. J. Appl. Phys. 44, 2860-2863
[Non-Patent Literature 2] T. Kajisa and T. Sakata, ChemElectroChem, 1, 1647-1655 (2014)
[Non-Patent Literature 3] T. Kajisa and T. Sakata, Jpn. J. Appl. Phys. 54, 04DL06 (2015)

### Summary of the Invention

### Problems to be Solved by the Invention

Ion-sensitive biosensors proposed thus far had a problem with noise arising from non-specific adsorption of substances etc. other than hydrogen ions (e.g. proteins such as albumin) to the electrode.

### Means for Solving the Problems

As a result of extensive investigation by the present inventors to reduce noise arising from non-specific adsorption in ion-sensitive biosensors, it was surprisingly found that noise arising from non-specific adsorption could be almost completely removed by coating the ion-sensitive electrode surface with polycatecholamine.

In other words, the present invention relates to an ion-sensitive biosensor comprising an ion-sensitive electrode, characterized in that all or a part of said ion-sensitive electrode surface is coated with polycatecholamine.

Moreover, one embodiment of the present invention is characterized in that said polycatecholamine is L-DOPA, dopamine, adrenaline, or noradrenaline polymer.

Moreover, one embodiment of the present invention is characterized in that the polycatecholamine coating on said ion-sensitive electrode surface is achieved by polymerizing catecholamine on the surface of said electrode.

Moreover, one embodiment of the present invention is characterized in that said ion-sensitive electrode is a gold electrode, a silver electrode, a copper electrode, a platinum electrode, an indium-tin oxide (ITO) electrode, a palladium electrode, a steel electrode, a nickel titanium alloy electrode, a titanium oxide electrode, a silicon dioxide electrode, a crystal electrode, an aluminum oxide electrode, a gallium arsenide electrode, a glass electrode, or a tantalum oxide electrode.

Moreover, one embodiment of the present invention is characterized in that said ion-sensitive electrode is hydrogen ion-sensitive.

Moreover, one embodiment of the present invention is characterized in that said ion-sensitive electrode is electrically connected to the gate electrode of a field effect transistor.

Moreover, one embodiment of the present invention is characterized in that said ion-sensitive electrode is placed away from said field effect transistor, and said ion-sensitive electrode is electrically connected to said gate electrode of said field effect transistor via electric wiring.

Moreover, one embodiment of the present invention is characterized in that said ion-sensitive electrode is electrically connected to said gate insulator film by being directly mounted on the gate insulator film of said field effect transistor.

Moreover, one embodiment of the present invention is characterized in that said ion-sensitive electrode is electrically connected to a signal amplifier.

Moreover, one embodiment of the present invention is characterized in that said signal amplifier is an operational amplifier.

Note that an invention of any combination of one or more characteristics of the present invention listed above is also encompassed by the scope of the present invention.

### Brief Description of the Drawings

Figure 1 shows a schematic diagram showing the outline configuration of the ion-sensitive biosensor which is one embodiment of the present invention.
Figure 2 shows the result of measuring the change in the gate surface potential of FET with the ion-sensitive biosensor which is one embodiment of the present invention, when test samples were added.
Figure 3 is a graph showing the amount of change in the gate surface potential of FET in the experimental result shown in Figure 2.
Figure 4 shows a graph showing the change in the gate surface potential of FET when a substance not targeted for detection (dopamine) was added to the electrode of the ion-sensitive biosensor which is one embodiment of the present invention.
Figure 5 shows a graph showing the change in the gate surface potential of FET when a substance not targeted for detection (albumin) was added to the electrode of the ion-sensitive biosensor which is one embodiment of the present invention.

### Description of Embodiments

The ion-sensitive sensor of the present invention is based on a basic principle to detect the change in pH of the test sample as the change in electrode charge density based on the change in ion concentration.

The ion-sensitive sensor of the present invention is characterized in that measurement noise arising from non-specific adsorption of contaminants in the test sample to the electrode is considerably reduced due to all or a part of the surface of the ion-sensitive electrode (the electrode for measuring the pH of the test sample) being coated with polycatecholamine. "Catecholamine" herein is a generic term for compounds that are induced from tyrosine and possesses a catechol and an amine, examples of which L-DOPA, dopamine, noradrenaline, adrenaline, and the like are known. Moreover, "polycatecholamine" means a catecholamine polymer, examples of which include L-DOPA, dopamine, noradrenaline, and adrenaline polymer.

The method for coating polycatecholamine on the electrode of the ion-sensitive electrode is not particularly limited, and can be appropriately selected by those skilled in the art. For example, since catecholamine is polymerized by oxidation, polymerization by auto-oxidation (air oxidation) can be induced by applying a catecholamine solution onto the ion-sensitive electrode and letting it stand still. Other oxidation methods include, e.g. electrochemical oxidation (such as cyclic voltammetry), UV ozone oxidation, addition of an oxidant such as potassium permanganate, and the like.

According to the present invention, pH can be accurately measured even in test samples comprising various contaminants. Accordingly, the pH of biologically derived samples, environmental samples, or samples in food, and the like can be measured with the biosensor of the present invention. Biologically derived samples that can be measured for pH with the biosensor of the present invention include, e.g., blood, lymph, tissue fluid, body cavity fluid, digestive juice, sweat, tear, nasal discharge, saliva, urine, seminal fluid, vaginal fluid, amniotic fluid, lactation, and the like.

### [Configuration of the ion-sensitive sensor]

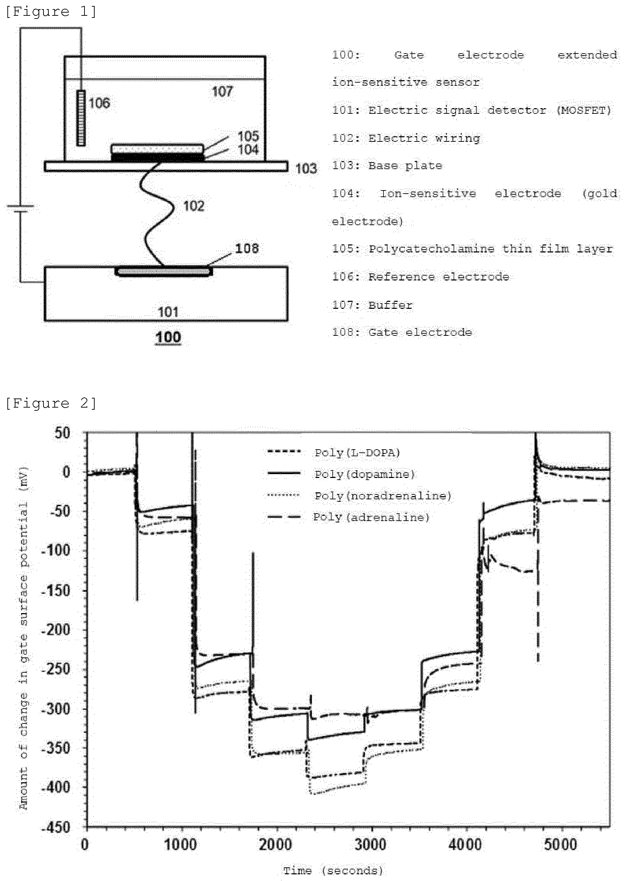

Figure 1 shows the sensor according to one embodiment of the present invention, and the constitution of the invention will be described. Figure 1 is a schematic diagram showing the outline configuration of ion-sensitive sensor 100 which is one embodiment of the present invention. Note that in the description below, description is made by way of an example when a so-called extended-gate FET is used as the detection element, but the ion-sensitive sensor according to the present invention is not to be limited to such an example. For example, an ordinary FET where the gate electrode is directly mounted on the insulator film may be employed.

Moreover, the ion-sensitive sensor according to the present invention is not limited to those employing FET as the detection element. The essential characteristic of the present invention is to detect the change in pH of the test sample as an electric signal in the ion-sensitive electrode portion coated with polycatecholamine, and for example, the said ion-sensitive electrode connected to a signal amplifier (such as a vacuum tube, a transistor, an operational amplifier, or a magnetic amplifier) can also be used as an ion-sensitive sensor.

As shown in Figure 1, ion-sensitive sensor 100 is a sensor that employs MOSFET (Metal Oxide Semiconductor Field Effect Transistor) 101 as the detection element for detecting the ion concentration in the test sample, and comprises ion-sensitive electrode 104 coated with polycatecholamine thin film layer 105. Ion-sensitive electrode 104 is sputtered onto base plate 105. Moreover, ion-sensitive electrode 104 is electrically connected to the gate electrode 108 of MOSFET 101 via electric wiring 102.

Moreover, on base plate 103 is fixed a glass ring so as to surround ion-sensitive electrode 104, and buffer 107 is filled inside the glass ring.

Note that as shown in Figure 1, reference electrode 106 may be provided as necessary. Reference electrode 106 is provided in buffer 107, and forms a closed circuit together with the source and drain electrodes of MOSFET 101. Reference electrode 106 is the electrode to be the reference potential for voltage measurement in FET, and may sometimes be grounded. In practice, Although it will be necessary for voltage measurement in FET, reference electrode 106 does not need to be provided if it can be substituted with another well-known method.

The semiconductor base plate of MOSFET 101 is for example a p-type semiconductor, and a part thereof (such as two places) is locally doped to form a n-type semiconductor portion on which the source and drain electrodes are provided. Note that the FET used in the ion-sensitive sensor of the present invention is not limited to the above n-channel MOSFET (n-MOS), and may be p-channel MOSFET (p-MOS), n-channel junction FET, or p-channel junction FET.

Moreover, the material for the semiconductor base plate is not particularly restricted, and well-known semiconductors such as Si, GaAs, transparent oxide semiconductors (such as ITO, IGZO, and IZO), organic semiconductors, and carbon semiconductors (such as carbon nanotube, graphene semiconductor, and diamond semiconductor etc.) can be appropriately selected and employed.

Moreover, the ion-sensitive sensor 100 which is one embodiment of the present invention uses the extended-gate FET as described above as the detection element. With an ion-sensitive sensor employing extended-gate FET, the ion-sensitive electrode portion is separated from the FET main body (FET 101 comprising a semiconductor base plate having source and drain electrodes provided thereon), and the ion-sensitive electrode portion can be freely detached and connected to FET 101.

In other words, in one embodiment of the present invention, the ion-sensitive electrode portion and the detection element (such as FET and signal amplifier) may be separately prepared and then combined. For example, the ion-sensitive electrode portion can also be configured as a detachable chip to the detection device main body (FET or signal amplifier).

The terms used herein, except for those that are particularly defined, are employed for describing particular embodiments, and do not intend to limit the invention.

Moreover, the term "comprising" as used herein, unless the content clearly indicates to be understood otherwise, intends the presence of the described items (such as components, steps, elements, and numbers), and does not exclude the presence of other items (such as components, steps, elements, and numbers).

Unless otherwise defined, all terms used herein (including technical and scientific terms) have the same meanings as those broadly recognized by those skilled in the art of the technology to which the present invention belongs. The terms used herein, unless explicitly defined otherwise, are to be construed as having meanings consistent with the meanings herein and in related technical fields, and shall not be construed as having idealized or excessively formal meanings.

The present invention will now be described in further detail with reference to Examples. However, the present invention can be embodied by various aspects, and shall not be construed as being limited to the Examples described herein.

### Examples

### Production of ion-sensitive biosensor having polydopamine thin film layer laminated onto gold electrode

The ion-sensitive sensor which is one embodiment of the present invention employed in the present Example was produced as follows (schematic diagram of the configuration is shown in Figure 1) .

In the ion-sensitive sensor employed in the present Example, MOSFET (from NXP, 2N7002) was employed as the detection element. As the electrode (ion-sensitive electrode) for detecting the charge of the target subject, a 6 mm diameter gold electrode sputtered onto a glass base plate was employed to produce a polydopamine thin film layer on the gold electrode by the method described below. Said gold electrode was set as an extended-gate electrode by electric connection via electric wiring from the gate electrode which is in direct contact with said MOSFET.

Next, in the present Example, in order to perform measurement in a solution, a glass ring having an outer diameter of 12 mm, an inner diameter of 10 mm, and a height of 10 mm was fixed onto the ion-sensitive electrode obtained as described above (the gold electrode coated with a polydopamine thin film layer) with epoxy resin.

### Production of polycatechol thin film layer on gold electrode

L-DOPA powder (D0600, Tokyo Chemical Industry Co., Ltd.), dopamine powder (A0305, Tokyo Chemical Industry Co., Ltd.), noradrenaline powder (A0906, Tokyo Chemical Industry Co., Ltd.), or adrenaline powder (A0173, Tokyo Chemical Industry Co., Ltd.) were each dissolved in Tris buffer (100 mM, pH 10) to produce 25 mM catecholamine solutions (L-DOPA solution, dopamine solution, noradrenaline solution, or noradrenaline solution). Next, 500 µl of one of the catecholamine solutions was placed in the aforementioned gold base plate having a glass ring fixed thereon, and a polycatecholamine thin film layer was formed on the gold base plate by irradiating UV for 5 minutes with an ultraviolet ray ozone surface treatment/reforming device (from MIZUKA PLANNING, PL-16) and then leaving at room temperature for 24 hours.

As a Comparative Example, a gold electrode (i.e. a gold electrode without coating with polycatecholamine) which has undergone a procedure similar to the above except that a simple Tris buffer (100 mM, pH10) was applied to the gold base plate having a glass ring fixed thereon was prepared.

### pH measurement of solution employing the sensor of the present invention

The ion-sensitive sensor produced was connected to a FET measurement device, and 500 µl of pH standard solutions at pH 1.68, 4.01, 7.41, 9.18, and 10.01 were replaced in the glass ring to detect the change in the gate surface potential in MOSFET when the solution pH changed.

The result of measuring the change in the gate surface potential employing the ion-sensitive sensor of the present invention when replaced with each pH standard solution is shown in Figure 2 The vertical axis in Figure 2 shows the change in surface potential (mV) of polycatecholamine, and the horizontal axis shows the measurement time (seconds).

As shown in Figure 2, it was shown that the ion-sensitive sensor of the present invention detects the change in pH with extreme acuity.

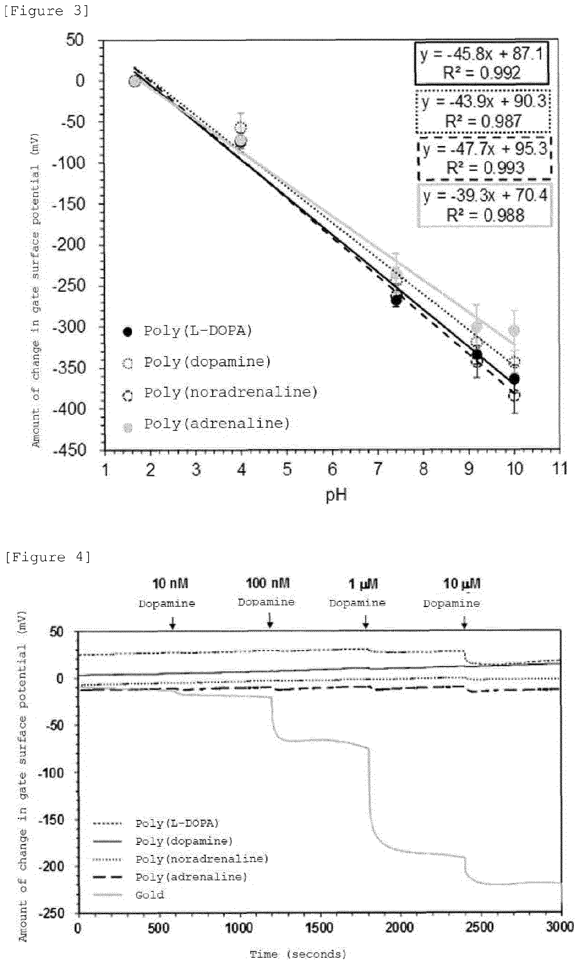

Moreover, the relationship between the range of change in the gate surface potential in Figure 2 and pH is shown in Figure 3. As shown in Figure 3, the ion-sensitive sensor of the present invention showed values of 39 - 48 mV/pH which are close to Nernst response, and variability was also low. In other words, it was shown that the ion-sensitive sensor of the present invention may function as a pH sensor with extreme accuracy.

In other words, it was shown that the ion-sensitive sensor of the present invention is a pH sensor superior in detection sensitivity and accuracy.

### Contaminant removal effect by polycatecholamine thin film layer

Next, a comparative experiment was performed in order to show that the ion-sensitive sensor of the present invention considerably reduces noise due to non-specific adsorption compared to an existing ion-sensitive sensor.

### (Comparative experiment employing dopamine as example of contaminant)

Five hundred microliters of sodium phosphate buffer (Phosphate buffered saline, PBS, pH 7.4) was placed in the glass ring of the ion-sensitive sensor of the present invention, and dopamine was added so that the final concentration will be 100 nM and 10 µm after gate potential stabilization.

As a Comparative Example, a similar test was performed with the ion-sensitive sensor having a gold electrode without coating with polycatecholamine produced by the method described above.

Note that the "dopamine" added to PBS in the present experiment was employed as an example of a contaminant (i.e. a substance that causes non-specific adsorption to the electrode) other than the detection target (hydrogen ion).

The result of the comparative experiment is shown in Figure 4. In Figure 4, the vertical axis shows the change in surface potential (mV) of polycatecholamine, and the horizontal axis shows the measurement time (seconds).

As shown in Figure 4, the ion-sensitive sensor of the present invention hardly showed any reaction to addition of dopamine (i.e. a substance not targeted for detection) in terms of the gate surface potential. On the other hand, the ion-sensitive sensor having a gold electrode without coating with polycatecholamine (Comparative Example) reacted to addition of dopamine and a negative potential shift was recognized.

### (Comparative experiment employing albumin as example of contaminant)

Five hundred microliters of sodium phosphate buffer (Phosphate buffered saline, PBS, pH 7.4) was placed in the glass ring of the ion-sensitive sensor of the present invention, and albumin was added so that the final concentration will be 1 g/L and 5 g/L after gate potential stabilization.

As a Comparative Example, a similar test was performed with the ion-sensitive sensor having a gold electrode without coating with polycatecholamine produced by the method described above.

Note that the "albumin" added to PBS in the present experiment was employed as an example of a contaminant (i.e. a substance that causes non-specific adsorption to the electrode) other than the detection target (hydrogen ion).

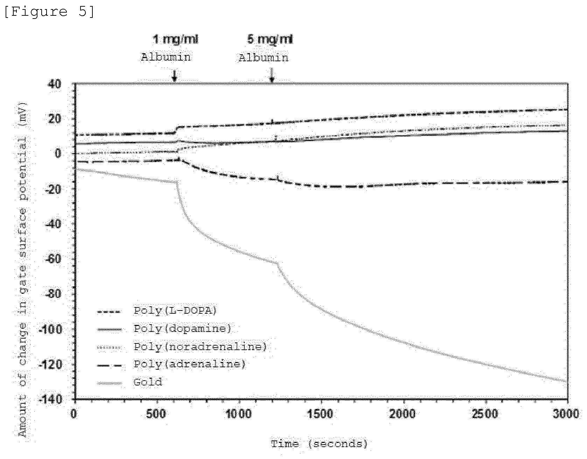

The result of the comparative experiment is shown in Figure 5. In Figure 5, the vertical axis shows the change in surface potential (mV) of polycatecholamine, and the horizontal axis shows the measurement time (seconds).

As shown in Figure 5, the ion-sensitive sensor of the present invention hardly showed any reaction to addition of albumin (i.e. a substance not targeted for detection) in terms of the gate surface potential. On the other hand, with the ion-sensitive sensor having a gold electrode without coating with polycatecholamine (Comparative Example), and a negative potential shift in reaction to addition of albumin was recognized.

From the above results, it was shown that the ion-sensitive sensor of the present invention inhibits the non-specific adsorption of substances not targeted for detection to the electrode, and that noise due to non-specific adsorption can be almost completely eliminated.

### Description of Symbols

- 100:: Gate electrode extended ion-sensitive sensor
- 101:: Electric signal detector (MOSFET)
- 102:: Electric wiring
- 103:: Base plate
- 104:: Ion-sensitive electrode (gold electrode)
- 105:: Polycatecholamine thin film layer
- 106:: Reference electrode
- 107:: Buffer
- 108:: Gate electrode

## Claims

1. An ion-sensitive biosensor comprising an ion-sensitive electrode, **characterized in that** all or a part of said ion-sensitive electrode surface is coated with polycatecholamine.

2. The biosensor according to claim 1, **characterized in that** said polycatecholamine is L-DOPA, dopamine, adrenaline, or noradrenaline polymer.

3. The biosensor according to claim 1, **characterized in that** the polycatecholamine coating on said ion-sensitive electrode surface is achieved by polymerizing catecholamine on the surface of said electrode.

4. The biosensor according to claim 1, **characterized in that** said ion-sensitive electrode is a gold electrode, a silver electrode, a copper electrode, a platinum electrode, an indium-tin oxide (ITO) electrode, a palladium electrode, a steel electrode, a nickel titanium alloy electrode, a titanium oxide electrode, a silicon dioxide electrode, a crystal electrode, an aluminum oxide electrode, a gallium arsenide electrode, a glass electrode, or a tantalum oxide electrode.

5. The biosensor according to claim 1, **characterized in that** said ion-sensitive electrode is hydrogen ion-sensitive.

6. The biosensor according to any one of claims 1 to 5, **characterized in that** said ion-sensitive electrode is electrically connected to the gate electrode of a field effect transistor.

7. The biosensor according to claim 6, **characterized in that**:
said ion-sensitive electrode is placed away from said field effect transistor, and
said ion-sensitive electrode is electrically connected to said gate electrode of said field effect transistor via electric wiring.

8. The biosensor according to claim 6, **characterized in that** said ion-sensitive electrode is electrically connected to said gate insulator film by being directly mounted on the gate insulator film of said field effect transistor.

9. The biosensor according to any one of claims 1 to 5, **characterized in that** said ion-sensitive electrode is electrically connected to a signal amplifier.

10. The biosensor according to claim 9, **characterized in that** said signal amplifier is an operational amplifier.
